# EUROPEAN PATENT SPECIFICATION

(11) **EP 3 634 445 B1**
(45) Date of publication and mention of the grant of the patent: **26.11.2025**
(21) Application number: 18735404.8
(22) Date of filing: 07.06.2018
(51) Int. Cl.: A61K 35/744, A61K 33/06, A61K 33/24, A61P 17/00, A61P 31/10, A61Q 5/00, A61K 8/11, A61K 8/19, A61K 8/99, A61K 33/26, A61K 47/14

(54) **COMBINATION AND TOPICAL COMPOSITIONS COMPRISING LACTIC BACTERIA AND METAL AND/OR SEMIMETAL OXIDES**
KOMBINATIONS- UND TOPISCHE ZUSAMMENSETZUNGEN MIT MILCHSÄUREBAKTERIEN UND METALL- UND/ODER HALBMETALLOXIDEN
COMBINAISON ET COMPOSITIONS TOPIQUES COMPRENANT DES BACTÉRIES LACTIQUES ET DES OXYDES MÉTALLIQUES ET/OU SEMI-MÉTALLIQUES

(30) Priority: 07.06.2017 IT 201700062260
(43) Date of publication of application: 15.04.2020
(73) Proprietor: Proge Farm S.r.l., 28100 Novara NO (IT)
(72) Inventor: MALFA, Patrizia, 28100 Novara NO (IT); DONDI, Giancarla, 28100 Novara NO (IT); COLOMBO, Giovanni Battista, 20060 Cassina de' Pecchi MI (IT)
(74) Representative: Trupiano, Federica
(86) International application number: PCT/IB2018/054086
(87) International publication number: WO 2018/224995

(56) References cited:
- EP-A1- 2 818 056
- WO-A1-2015/173693
- WO-A1-2015/175333
- WO-A1-2016/113365
- WO-A1-2017/087640
- JP-A- H0 286 770
- ERFAN KHERADMAND ET AL: "The antimicrobial effects of selenium nanoparticle-enriched probiotics and their fermented broth against Candida albicans", DARU JOURNAL OF PHARMACEUTICAL SCIENCES, BIOMED CENTRAL LTD, LONDON, UK, vol. 22, no. 1, 6 June 2014 (2014-06-06), pages 48, XP021188721, ISSN: 2008-2231, DOI: 10.1186/2008-2231-22-48
- A GUENICHE ET AL: "oral supplementation with probiotic lactobacillus parasei improves dandruff condition", 15 July 2011 (2011-07-15), XP055443458, Retrieved from the Internet <URL:https://www.ncbi.nlm.nih.gov/pmc/articles/PMC3171853/?report=printable> [retrieved on 20180123]
- KUANG HUIJUAN ET AL: "Synergisticin vitroandin vivoantimicrobial effect of a mixture of ZnO nanoparticles andLactobacillusfermentation liquor", APPLIED MICROBIOLOGY AND BIOTECHNOLOGY, SPRINGER, DE, vol. 100, no. 8, 23 December 2015 (2015-12-23), pages 3757 - 3766, XP035870798, ISSN: 0175-7598, [retrieved on 20151223], DOI: 10.1007/S00253-015-7221-X
- J.-J. NAM ET AL: "Metal oxide-coating PMMA or Talc as a new IR blocker inhibits IR-induced decrease of collagens in human dermal fibroblasts", INTERNATIONAL JOURNAL OF COSMETIC SCIENCE., vol. 37, no. 4, 13 April 2015 (2015-04-13), NL, pages 433 - 437, XP055323864, ISSN: 0142-5463, DOI: 10.1111/ics.12214
- CHIROM AARTI ET AL: "In vitro investigation on probiotic, anti- Candida , and antibiofilm properties of Lactobacillus pentosus strain LAP1", ARCHIVES OF ORAL BIOLOGY., vol. 89, 23 February 2018 (2018-02-23), GB, pages 99 - 106, XP055500154, ISSN: 0003-9969, DOI: 10.1016/j.archoralbio.2018.02.014
- NETTIS E ET AL: "Probiotics and refractory chronic spontaneous urticaria.", EUROPEAN ANNALS OF ALLERGY AND CLINICAL IMMUNOLOGY SEP 2016, vol. 48, no. 5, September 2016 (2016-09-01), pages 182 - 187, XP009507477, ISSN: 1764-1489

## Description

### ABSTRACT

The present invention relates to a topical combination and topical compositions comprising lactobacilli and metal and/or semimetal oxides, useful in the prophylaxis and/or treatment of cutaneous and mucosal diseases caused by *Malassezia* fungi, as well as their use in the therapy of said diseases.

### STATE OF THE ART

Recently it has been observed that diseases caused by the proliferation of pathogenic microorganisms on the skin and the mucous membranes can be treated by the topical use, i.e. directly on the interested part, of non-pathogenic bacteria, such as for example the probiotic bacteria, which colonize the skin or mucosa portion on which they are applied, hindering and often completely solving the infection in place.

The treatment or prophylaxis of said skin diseases by the topical use of probiotic bacteria has some advantages with respect to the conventional topical treatment with antimicrobials; for example, by the use of the latter in determined conditions, the well-known phenomenon of antimicrobial resistance can occur.

The topical compositions comprising probiotic bacteria proved to be useful not only for the cutaneous diseases caused by pathogenic microorganisms, such as pityriasis versicolor, but it has been observed that they also allow the prevention and treatment of different cutaneous and mucosal diseases, such as for example dandruff, various types of dermatitis, candidiasis and the like.

Notwithstanding the topical compositions comprising probiotic bacteria are relatively novel and promising compositions, able to effectively treating a great number of cutaneous and mucosal diseases, however there is the need to research for improved compositions, in order to increase their prophylactic and therapeutic effect towards the above mentioned diseases and possibly to widen their field of application.

### OBJECTS OF THE INVENTION

It is an object of the present invention to provide a combination consisting of probiotic bacteria, to be used by topical route, and having better performance with respect to the conventional compositions comprising probiotic bacteria.

It is also an object of the present invention to provide a composition comprising said combination, allowing comfortably applying the combination at topical level and not hindering, but possibly improving, the performances of said combination.

It is also an object of the present invention to provide a use of said composition in the prophylaxis and treatment of cutaneous and mucosal diseases caused by *Malassezia* fungi.

### DESCRIPTION OF THE INVENTION

According to one of its aspects, it is a subject-matter of the present invention a topical composition which comprises a combination consisting of:
a) one or more probiotic bacteria selected from *Lactobacillus crispatus* P 17631, *Lactobacillus paracasei* I 1688 and mixtures thereof; and
b) one or more pharmaceutically acceptable metal oxides and/or one or more pharmaceutically acceptable semimetal oxides selected from Al₂O₃, Fe₂O₃, TiO₂, Cr₂O₃, MgO, ZrO₂, MnO₂, Co₂O₃ and Y₂O₃, together with conventional excipients and/or carriers, and preferably also colloidal anhydrous silica.
for its use in the topical prevention and/or treatment of cutaneous and mucosal diseases caused by *Malassezia* fungi

The expression "one or more pharmaceutically acceptable metal oxides and/or one or more pharmaceutically acceptable semimetal oxides" comprises both the oxides as such, and in the modified form.

The "modified" oxides of the invention are oxides that have been subjected to a calcination treatment. The calcination is a treatment known to the person skilled in the art and provides for some compounds, in the specific case the oxides as herein defined, being subjected to heat treatment according to temperature gradients ranging from 1200°C to 1600°C, so to generate a partial calcination and the consequent modification of the crystallographic conformation of the starting oxides, that can be verified by mass spectrophotometry.

It is evident that mixtures of bacteria a) and mixture of metal oxides and semimetal oxides b) can be used according to the invention.

The expression "pharmaceutically acceptable" means that said oxides are neither toxic nor in any way harmful to the organism and can be applied on the skin without causing harmful side effects.

According to an embodiment, the combination of the invention is a fixed combination, and the weight ratio between said one or more bacteria and said one or more metal oxides and/or one or more semimetal oxides is 50-5/1, preferably 40-10/1, more preferably 30-15/1, even more preferably 20/1.

Unless otherwise stated, it is herein considered that 1 gram of bacteria contains from about 1x 10³ to 1x 10¹¹ UFC, preferably from 1x 10⁵ to 1x 10⁹ UFC, more preferably from 1x 10 ⁶ to 1x 10 ⁸ UFC.

It has been surprisingly observed that the combination of the invention has particular mechanical properties allowing to trigger surprising prophylactic and/or therapeutic properties against the cutaneous and mucosal diseases.

By mechanical properties is herein meant the capability to adhere and colonize the tissue by the bacteria on the skin and the mucous membranes.

It is known that bacteria inserted in a micro-environment having characteristics that are not favorable for them, will show poorly satisfactory efficacy, such as for example for the reduced adhesion involved by a poor production of metabolites, and the like. Vice versa, if the micro-environment is suitable to their growth, probiotic bacteria can adequately exert their antagonist action against pathogenic bacteria.

The present Applicant conceived to optimize said micro-environment that in the present case is represented by the skin and the mucous membrane, so that probiotic bacteria can exert the mechanical properties allowing to trigger improved prophylactic and therapeutic activity against cutaneous diseases. In this way, the probiotic bacteria will show better capability to adhere, more effective metabolism and higher proliferation capability with respect to the same probiotic bacteria inserted in an inhospitable micro-environment.

It is known that the skin surface has average temperature of about 25°C, which can naturally vary based on the temperature of the external environment. Such a temperature is certainly lower than the favorable temperatures of the culture media suitable for the proliferation of the probiotic bacteria such as those mentioned above, that is around 37±2°C. Therefore, probiotic bacteria administered by topical route through a conventional composition, for example on the skin, find an unsuitable micro-environment that determines stress of the bacteria themselves, because of the temperature that is poorly suitable to their proliferation.

It has been surprisingly found that the combination of the invention, when applied at topical level, i.e. on the skin and the mucous membranes, allows a suitable micro-environment towards the bacteria contained therein to be created, as the temperature of the application area is higher than the non-treated areas and, like this, the temperature of the micro-environment is more similar to the ideal temperature for the proliferation of said bacteria.

While not willing to be bound to any theory, it is hypothesized that such a temperature increase is due to the presence of said one or more metal oxides and/or one or more semimetal oxides. Premise that all of the bodies having temperature higher than the absolute zero, including the human body, emit infrared rays and that infrared rays are electromagnetic radiations heating the bodies they hit, it is supposed that the metal and semimetal oxides of the combination of the invention allow keeping the infrared rays emitted by the human body on the surface of the same, thus causing the heating of the area hit by the infrared rays, i.e. the cutaneous/mucosal application area of the combination of the invention. Presumably, said metal and semimetal oxides act by the reflection of the infrared rays emitted by the human body, likely, in addition to the emission of infrared rays. In other words, it would seem that said metal and semimetal oxides avoid the dispersion of the heat emitted by the part of the body that has been treated, acting as a kind of reflecting barrier.

Therefore it is understood that the combination of the invention, by allowing to optimize the micro-environment on which it is administered, enables to make the most out of the prophylactic and therapeutic characteristics of the bacteria contained therein and therefore making them more effective against the cutaneous diseases, in particular, but not only, those caused by pathogenic microorganisms.

As already mentioned and as it will be demonstrated in the Experimental Section, the combination of the invention is useful for the prevention and/or treatment of cutaneous diseases caused by the fungi of the *Malassezia* genus, such as pityriasis versicolor, seborrheic dermatitis and/or dandruff.

As it will be shown in the following Experimental Section, in a study aimed at evaluating the effectiveness of the combination in the treatment of the cutaneous injuries associated to the depigmentation by *Malassezia,* it has been demonstrated the capability of the combination to create a colonizing and protective effect on the tested tissue structure and to stimulate the proliferation of melanocytes with an increase of the amount of produced pigment and complete remission of the disease.

The combination has also showed being more effective than the treatment with ketoconazole in the treatment of fungal infections.

By "combination" is herein meant to denote a mixture consisting of one or more probiotic bacteria as herein defined and one or more pharmaceutically acceptable metal oxides and/or one or more pharmaceutically acceptable semimetal oxides. For the preparation of the mixture it is sufficient to mix the probiotic bacteria, preferably in a lyophilized form, and the desired oxides.

Such a combination can be administered topically to a human subject alone or, preferably, in the form of compositions for topical use together with conventional vehicles and/or excipients.

According to another of its aspects, it is also a subject-matter of the present invention a topical composition characterized in that it comprises the combination of the invention together with conventional vehicles and excipients.

The topical composition of the invention can be any conventional topical composition formulated and/or set up by equally known processes. For example, the topical composition of the invention can be selected from creams, multiple emulsions such as oil and/or silicone in water emulsions, water-in-oil and/or -silicone emulsions, water/oil/water or water/silicone/water emulsions and oil/water/oil or silicone/water/silicone emulsions, anhydrous compositions, aqueous dispersions, oils, milk, balms, foams, lotions, gel, cream gel, hydroalcoholic solutions, hydroglycolic solutions, hydrogel, liniments, waxes, soaps, shampoos, emollients, serums, ointments, mousses, ointments, powders, pastes, spray and others. The composition can also be administered by polysaccharide films, sticking patches, non-sticking patches, occlusive patches, and micro-electric patches.

Alternatively, the composition of the invention, as well as the combination of the invention, can be incorporated in a solid accessory, for example selected from bandages, gauzes, t-shirts, socks, tights, linen, belts, gloves, nappies, pads, garments, bedcover, wipes, facial masks; the incorporation of the composition or combination of the invention in a solid accessory allows the ease of application of the composition or combination of the invention and also, in some embodiments, such as for example when the composition or the combination of the invention is incorporated in t-shirts, enables the same to adhere on a wide body surface, which is useful when wide skin areas, such as the back, must be treated for example to prevent and/or treat the pityriasis versicolor.

Alternatively, the combination can be included in cosmetic products such as foundation creams, lotions for make-up removal, milk for make-up removal, concealer for periorbital dark circles, eye shadows, lipsticks, lip protectors, lip glosses and the like. Said incorporation can be carried out by the conventional methods, well-known to the person skilled in the art.

In a preferred embodiment, the composition of the invention is characterized in that it comprises colloidal anhydrous silica. The colloidal anhydrous silica is conventionally used as gelling and stabilizing agent, but is also useful in the composition of the invention as it confers exfoliating capabilities to the composition of the invention. In fact the colloidal anhydrous silica, when it is in a suspension, allows the composition of the invention having a peeling effect, i.e. a smoothing effect on the skin, which is useful to remove its superficial dead cells, thus allowing the probiotic bacteria included in the composition of the invention better colonizing the part and more easily reaching the pathogenic microorganisms that can reside in the layers of the epidermis.

The composition of the invention can be included or incorporated in mono-dose containers or multi-dose containers. For example, the composition of the invention can be an ointment divided in mono-dose containers, for example inside capsules, advantageously mono-dose squeezable capsules.

The amount of composition inside the mono-dose container can vary as a function of the area to be treated. Consequently, different mono-dose containers containing different amounts of composition can be provided.

The composition of the invention is preferably an occlusive composition, such as for example an ointment. This allows the skin keeping a higher water amount, and consequently creating an even more favorable micro-environment for the proliferation of the probiotic bacteria. Furthermore, the composition of the invention in the form of ointment allows the application on a wide skin surface even with small volumes of the composition itself.

The bacteria and the metal or semimetal oxides used according to the present invention are not toxic nor in any way harmful. For this reason, the amounts of lactobacilli and oxides are not critical and can also vary as a function of the area to be treated.

By way of example, a representative mono-dose composition of the invention can contain an amount of probiotic bacteria described above in the range from 0.05 g to 5 g, preferably from 0.5 g to 1.5 g, more preferably about 1 g, and an amount of metal and/or semimetal oxides in the range from 10 mg to 100 mg, preferably from 40 mg to 60 mg, more preferably about 50 mg, still respecting the proportions of the combination of the invention mentioned above.

According to an embodiment, said representative mono-dose composition of the invention can comprise a weight amount of probiotic bacteria and metal/semimetal oxides in the range from 10% to 30%, preferably about 20%, with respect to the weight of said composition.

Some representative examples of compositions according to the invention are set forth in the following experimental section.

It is evident that the composition of the invention has the same uses of the combination of the invention. Therefore, objects of the present invention are the combination and the composition of the invention for their medical and/or cosmetic use, preferably for their use in the prevention and/or treatment of the cutaneous and mucosal diseases mentioned above and, more preferably, for their use in the prevention and/or treatment of pityriasis versicolor and/or seborrheic dermatitis.

In general, in order to exert the beneficial effect against the pathogenic microorganisms, the composition is applied on the skin and the mucous membranes from 1 or several times a day, better if it's applied on wet skin, being preferably applied on a part wider than the visually affected part, in order to ensure the whole part interested by the disease is covered. The treatment can last 3 to 15 days, for example 3-7 days. If necessary, the treatment can be further prolonged or periodically repeated.

It is a further object of the invention a method for the prevention and/or treatment of the cutaneous diseases mentioned above, comprising topically administering an effective amount of the combination or composition of the invention as depicted above, to a subject in the need thereof.

The invention also concerns the use of metal oxides and/or semimetal oxides to generate a micro-environment in which the probiotic bacteria show optimal adhesion capabilities, effective metabolism and high proliferation capability. Therefore, it is also a subject-matter of the invention the use of metal oxides and/or semimetal oxides to favor the proliferation and activity of probiotic bacteria in topical compositions.

The preferred and advantageous aspects of the invention set forth above with reference to the combination are also considered applicable to the composition and the treatment method according to the invention.

The following Experimental Section is set forth only by way of illustration of the invention and in no way as a limitation.

### BRIEF DESCRIPTION OF THE FIGURES

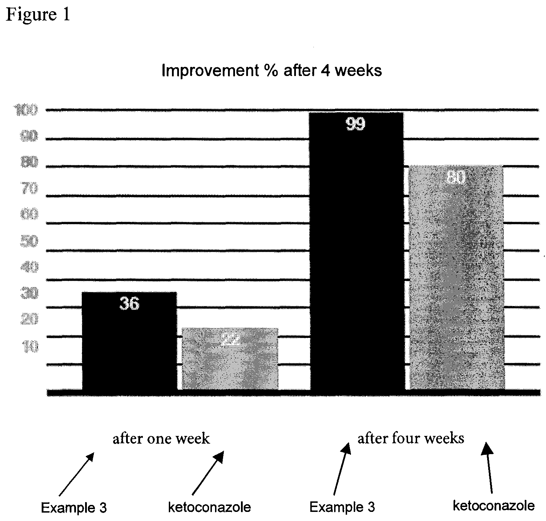

Figure 1 shows the improvement percentage after four weeks in the patients treated as described in Example 4.

### EXPERIMENTAL SECTION

### Example 1

### Quali-quantitative composition of a squeezable capsule containing the composition of the invention in the form of ointment

An embodiment of the composition of the invention has been made in the form of ointment and divided in mono-dose squeezable capsules of 1.2 ml. Each capsule has the amounts of components shown in Table 1 (the percentages of the composition are referred to the weight percentages to the total weight of the composition).

**Table 1a**

| COMPOSITION | |
|---|---|
| **OINTMENT** | **%** |
| Medium chain triglycerides | 75.875 |
| Anhydrous colloidal silica | 3.50 |
| Iron oxides | 0.025 |
| Mixture of lactobacilli and modified metal oxides in 20:1 ratio (w/w) | 20.00 |
| Flavor | 0.60 |

CONTAINER: squeezable capsule; capsule format: 20 TOT K; capsule casing color: CL 3386 (MATT BROWN).

### Example 2

### Quali-quantitative composition of a squeezable capsule containing the composition of the invention in the form of ointment

An embodiment of the composition of the invention has been made in the form of ointment and divided in mono-dose squeezable capsules of 1.2 ml. Each capsule has the amounts of components shown in Table 1 (the percentages of the composition are referred to the weight percentages to the total weight of the composition).

**Table 1b**

| COMPOSITION | |
|---|---|
| **OINTMENT** | **%** |
| Medium chain triglycerides | 75.875 |
| Anhydrous colloidal silica | 3.50 |
| Iron oxides | 0.025 |
| Mixture of lactobacilli and non-modified metal oxides in 20:1 ratio (w/w) | 20.00 |
| Flavor | 0.60 |

CONTAINER: squeezable capsule; capsule format: 20 TOT K; capsule casing color: CL 3386 (MATT BROWN).

### Example 3

### Quali-quantitative composition of a squeezable capsule containing the composition of the invention in the form of ointment

An embodiment of the composition of the invention has been made in the form of ointment and divided in mono-dose squeezable capsules of 1.2 ml. Each capsule has the amounts of components shown in Table 1 (the percentages of the composition are referred to the weight percentages to the total weight of the composition).

**Table 1c**

| COMPOSITION | |
|---|---|
| **OINTMENT** | **%** |
| Medium chain triglycerides | 75.875 |
| Anhydrous colloidal silica | 3.50 |
| Iron oxides | 0.025 |
| Mixture of *Lactobacillus crispatus* P 17631 and *Lactobacillus paracasei* I 1688/ modified manganese dioxide and iron III oxide in a 20/1 ratio (w/w) | 20.00 |
| Flavor | 0.60 |

CONTAINER: squeezable capsule; capsule format: 20 TOT K; capsule casing color: CL 3386 (MATT BROWN).

### Example 4

### Quali-quantitative composition of a squeezable capsule containing the composition of the invention in the form of ointment

An embodiment of the composition of the invention has been made in the form of ointment and divided in mono-dose squeezable capsules of 1.2 ml. Each capsule has the amounts of components shown in Table 1 (the percentages of the composition are referred to the weight percentages to the total weight of the composition).

**Table 1d**

| COMPOSITION | |
|---|---|
| **OINTMENT** | **%** |
| Medium chain triglycerides | 75.875 |
| Anhydrous colloidal silica | 3.50 |
| Iron oxides | 0.025 |
| Mixture of *Lactobacillus salivarius* LS01 (DSM 22775) strain, *Bifidobacterium breve* BR03 (DSM 16604) and *Lactobacillus pentosus* (DSM 21980) / modified manganese dioxide and iron III oxide in a 15/1 ratio (w/w) | 20.00 |
| Flavor | 0.60 |

CONTAINER: squeezable capsule; capsule format: 20 TOT K; capsule casing color: CL 3386 (MATT BROWN).

### Example 5

The manufacturing process carried out for producing the squeezable capsules according to Example 1 is the following: the raw materials described in Table 1 are weighted on analytical electronic scales, whereupon proceed in parallel by the following processes a) and b):
a) the fat mixture of the ointment (medium chain triglycerides) comprising colloidal anhydrous silica is prepared, whereupon the mixture of probiotic bacteria and metal and semimetal oxides included in the combination of the invention is dispersed in it; finally, the mass is deaerated;
b) the mass that will constitute the squeezable capsule is prepared according to conventional methods, whereupon the mass is unloaded.

The ointment prepared by the process a) is encapsulated inside the squeezable capsule prepared by the process b) by the encapsulating machine, whereupon the primary drying and the secondary drying at room temperature are sequentially carried out.

### Example 6

### Test on the therapeutic and prophylactic effectiveness

*In vivo* tests have been carried out on 8 volunteers that have presented relapses of pityriasis versicolor every year: in fact the patients showed yearly small light spots on the back and the arms, due to the destruction of the melanocytes carried out by the *Malassezia* fungus.

The *in vivo* tests provided the treatment of pityriasis versicolor by the application of the composition of Example 1, prepared according to the process of Example 3, by employing the composition once a day. Said composition has been homogeneously applied both on the affected skin surface (i.e., the skin surface having the light spots), and in the areas adjacent to it, in order to prevent the development of *Malassezia* colonies not yet visible.

In particular, the composition has been applied by two different strategies:
- on 4 volunteers (group A), it has been applied on the dry skin, by exerting a light scrub effect, thus allowing to remove the dead cells of the epidermis and the subsequent colonization by the probiotic microorganisms that are in the composition;
- on the remaining 4 volunteers (group B), it has been applied on the wet skin, by limiting the scrub effect but increasing the spreadability of the composition.

After some applications already, more precisely from 2 to 3 applications, a block of the replication of *Malassezia* in the volunteers of group A has been observed, as well as some symptom improvements (absence of itching and desquamation). In the volunteers of the group B the same effects have been observed from 4 applications onwards. For the volunteers of both groups, after 15 and 30 days from the first treatment, also the dyschromia has been improved, as there have been strong improvements after the solar exposure, already emerged by the improved fluorescence following the evaluation under the Wood light.

Finally, one year after the treatment, for both patient groups it has been observed that the spots typical of pityriasis versicolor did not reappear in the treated areas.

The results of the present *in vivo* test confirm the capability of the combination and composition of the invention to effectively treat and prevent the cutaneous diseases, showing surprising effects also one year after the treatment, thus demonstrating their capability in eradicating the colonies of pathogenic microorganisms from the skin surface, such as that of the *Malassezia* fungus.

### Example 7

### Comparative test with respect to ketoconazole in the treatment of pityriasis versicolor

A prospective randomized study was carried out in patients having typical lesions of pityriasis versicolor that have been confirmed by the direct microbiological culture examination and by the examination under the Wood light.
- 10 patients treated with the composition of Example 3, once a day for a week;
- 10 patients treated with ketoconazole foam, once a day for a week.

The clinical response has been evaluated based on the visual clinical (photographic) and mycological examinations collected every week for the entire follow-up period of one month. The patients did not report adverse reactions during the use of the composition of Example 3.

At the end of the follow-up period, the improvement induced by the therapy with the composition of Example 3 was statistically significant (p<0.05) with respect to that obtained with ketoconazole foam (Figure 1).

## Claims

1. A topical composition which comprises a combination consisting of:
a) one or more probiotic bacteria selected from *Lactobacillus crispatus* P 17631, *Lactobacillus paracasei* I 1688 and mixtures thereof; and
b) one or more pharmaceutically acceptable metal oxides and/or one or more pharmaceutically acceptable semimetal oxides selected from Al₂Os, Fe₂0₃, TiO₂, Cr₂O₃, MgO, ZrO₂, MnO₂, Co₂O₃ and Y₂O₃, together with conventional excipients and/or carriers, and preferably also colloidal anhydrous silica,
for its use in the topical prevention and/or treatment of cutaneous and mucosal diseases caused by *Malassezia* fungi.

2. The composition for use according to claim 1, in the treatment of pityriasis versicolor, seborrheic dermatitis and/or dandruff.

3. The composition for use according to claim 1 or 2, **characterized in that** said one or more pharmaceutically acceptable metal oxides and/or one or more pharmaceutically acceptable semimetal oxides have been subjected to calcination.

4. The composition for use according to any one of the preceding claims, **characterized in that** said one or more pharmaceutically acceptable metal oxides and/or one or more pharmaceutically acceptable semimetal oxides are selected from MnO₂ and Fe₂O₃.

5. The composition for use according to any one of the preceding claims, **characterized in that** said one or more bacteria / said one or more metal oxides and/or one or more semimetal oxides weight ratio is 50-5/1, preferably 40-10/1, more preferably 30-15/1, even more preferably 20/1.

6. The composition for use according to any one of the preceding claims, **characterized in that** it is selected from: creams, multiple emulsions such as oil and/or silicone in water emulsions, water-in-oil and/or -silicone emulsions, water/oil/water or water/silicone/water emulsions and oil/water/oil or silicone/water/silicone emulsions, anhydrous compositions, aqueous dispersions, oils, milk, balms, foams, lotions, gel, cream gel, hydroalcoholic solutions, hydroglycolic solutions, hydrogel, liniments, waxes, soaps, shampoos, emollients, serums, polysaccharide films, ointments, mousses, ointments, powders, sticking patches, non-sticking patches, occlusive patches, micro-electric patches, pastes and sprays.

7. The composition for use according to claim 5, **characterized in that** it is incorporated into a solid accessory selected from: bandages, gauzes, t-shirts, socks, tights, linen, belts, gloves, nappies, pads, garments, bedcover, wipes, facial masks, foundation creams, lotions for make-up removal, milk for make-up removal, concealer for periorbital dark circles, eye shadows, lipsticks, lip protectors and lip glosses.

## Patentansprüche

1. Eine topische Zusammensetzung, die eine Kombination umfasst, bestehend aus:
a) einem oder mehreren probiotischen Bakterien, ausgewählt aus *Lactobacillus crispatus* P 17631, *Lactobacillus paracasei* I 1688 und deren Mischungen; und
b) einem oder mehreren pharmazeutisch verträglichen Metalloxiden und/oder einem oder mehreren pharmazeutisch verträglichen Halbmetalloxiden, ausgewählt aus Al₂O₃, Fe₂O₃, TiO₂, Cr₂O₃, MgO, ZrO₂, MnO₂, Co₂O₃ und Y₂O₃, zusammen mit herkömmlichen Hilfsstoffen und/oder Trägerstoffen, und vorzugsweise auch mit kolloidalem wasserfreiem Siliciumdioxid,
für ihre Verwendung zur topischen Vorbeugung und/oder Behandlung von kutanen und mukosalen Erkrankungen, die durch Malassezia-Pilze verursacht werden.

2. Die Zusammensetzung für Verwendung nach Anspruch 1 zur Behandlung von Pityriasis versicolor, seborrhoischem Dermatitis und/oder Schuppen.

3. Die Zusammensetzung für Verwendung nach Anspruch 1 oder 2, **dadurch gekennzeichnet, dass** das ein oder die mehreren pharmazeutisch verträglichen Metalloxide und/oder das ein oder die mehreren pharmazeutisch verträglichen Halbmetalloxide einer Kalzinierung unterzogen wurden.

4. Die Zusammensetzung für Verwendung nach einem der vorhergehenden Ansprüche, **dadurch gekennzeichnet, dass** das ein oder die mehreren pharmazeutisch verträglichen Metalloxide und/oder das ein oder die mehreren pharmazeutisch verträglichen Halbmetalloxide aus MnO₂ und Fe₂O₃ ausgewählt sind.

5. Die Zusammensetzung für Verwendung nach einem der vorhergehenden Ansprüche, **dadurch gekennzeichnet, dass** das Gewichtsverhältnis des einen oder der mehreren Bakterien zu dem einen oder den mehreren Metalloxiden und/oder dem einen oder den mehreren Halbmetalloxiden 50-5/1 beträgt, vorzugsweise 40-10/1, besonders bevorzugt 30-15/1, noch bevorzugter 20/1.

6. Die Zusammensetzung für Verwendung nach einem der vorhergehenden Ansprüche, **dadurch gekennzeichnet, dass** sie ausgewählt ist aus: Cremen, Mehrfachemulsionen wie Öl- und/oder Silikon-in-Wasser-Emulsionen, Wasserin-Öl- und/oder -Silikon-Emulsionen, Wasser/Öl/Wasser- oder Wasser/Silikon/Wasser-Emulsionen und Öl/Wasser/Öl- oder Silikon/Wasser/Silikon-Emulsionen, wasserfreien Zusammensetzungen, wässrigen Dispersionen, Ölen, Milch, Balsamen, Schäumen, Lotionen, Gelen, Cremegelen, hydroalkoholischen Lösungen, hydroglykolischen Lösungen, Hydrogelen, Linimenten, Wachsen, Seifen, Shampoos, Emollientien, Seren, Polysaccharidfilmen, Salben, Moussen, Salben, Pulver, haftenden Pflastern, nicht haftenden Pflastern, okklusiven Pflastern, mikroelektrischen Pflastern, Pasten und Sprays.

7. Die Zusammensetzung für Verwendung nach Anspruch 5, **dadurch gekennzeichnet, dass** sie in ein festes Zubehör integriert ist, ausgewählt aus: Bandagen, Gazen, T-Shirts, Socken, Strumpfhosen, Wäsche, Gürteln, Handschuhen, Windeln, Einlagen, Kleidungsstücken, Bettdecken, Tüchern, Gesichtsmasken, Foundation-Cremen, Lotionen zum Abschminken, Reinigungsmilch, Concealern gegen Augenringe, Lidschatten, Lippenstiften, Lippenpflegeprodukten und Lipglossen.

## Revendications

1. Composition topique qui comprend une combinaison consistant en :
a) une ou plusieurs bactéries probiotiques choisies parmi *Lactobacillus crispatus* P 17631, *Lactobacillus paracasei* I 1688 et leurs mélanges ; et
b) un ou plusieurs oxydes métalliques pharmaceutiquement acceptables et/ou un ou plusieurs oxydes semi-métalliques pharmaceutiquement acceptables choisis parmi Al₂O₃, Fe₂O₃, TiO₂, Cr₂O₃, MgO, ZrO₂, MnO₂, Co₂O₃ et Y₂O₃, ainsi que des excipients et/ou des supports conventionnels, et de préférence aussi de la silice colloïdale anhydre,
pour son utilisation dans la prévention et/ou le traitement topique des maladies cutanées et muqueuses causées par les champignons *Malassezia*.

2. Composition pour utilisation selon la revendication 1, dans le traitement du pityriasis versicolor, de la dermatite séborrhéique et/ou des pellicules.

3. Composition pour utilisation selon la revendication 1 ou 2, **caractérisée en ce que** ledit un ou plusieurs oxydes métalliques pharmaceutiquement acceptables et/ou un ou plusieurs oxydes semi-métalliques pharmaceutiquement acceptables ont été soumis à une calcination.

4. Composition pour utilisation selon l'une quelconque des revendications précédentes, **caractérisée en ce que** ledit ou lesdits oxydes métalliques pharmaceutiquement acceptables et/ou ledit ou lesdits oxydes semi-métalliques pharmaceutiquement acceptables sont choisis parmi MnO₂ et Fe₂O₃.

5. Composition pour utilisation selon l'une quelconque des revendications précédentes, **caractérisée en ce que** ledit rapport pondéral une ou plusieurs bactéries / un ou plusieurs oxydes métalliques et/ou un ou plusieurs oxydes semi-métalliques est de 50-5/1, de préférence 40-10/1, plus préférentiellement 30-15/1, encore plus préférentiellement 20/1.

6. Composition pour utilisation selon l'une quelconque des revendications précédentes, **caractérisée en ce qu'**elle est choisie parmi : les crèmes, les émulsions multiples telles que les émulsions huile et/ou silicone dans l'eau, les émulsions eau dans l'huile et/ou silicone, les émulsions eau/huile/eau ou eau/silicone/eau et les émulsions huile/eau/huile ou silicone/eau/silicone, les compositions anhydres, les dispersions aqueuses, les huiles, les laits, les baumes, les mousses, les lotions, le gel, le gel crème, les solutions hydroalcooliques, les solutions hydroglycoliques, le hydrogel, les liniments, les cires, les savons, les shampooings, les émollients, les sérums, les films de polysaccharides, les pommades, les mousses, les pommades, les poudres, les patchs collants, les patchs non collants, les patchs occlusifs, les patchs micro-électriques, les pâtes et les sprays.

7. Composition pour utilisation selon la revendication 5, **caractérisée en ce qu'**elle est incorporée dans un accessoire solide choisi parmi : les bandages, les gazes, les t-shirts, les chaussettes, les collants, le linge, les ceintures, les gants, les couches, les tampons, les vêtements, les couvre-lits, les lingettes, les masques faciaux, les crèmes de fond de teint, les lotions démaquillantes, les laits démaquillants, les correcteurs de cernes périorbitaires, les ombres à paupières, les rouges à lèvres, les protecteurs de lèvres et les brillants pour les lèvres.
